Europäisches Patentamt

European Patent Office

Office européen des brevets

(19)

(11) **EP 0 858 797 B1**

(12) **FASCICULE DE BREVET EUROPEEN**

(45) Date de publication et mention
de la délivrance du brevet:
**31.07.2002 Bulletin 2002/31**

(51) Int Cl.⁷: **A61K 7/32**

(21) Numéro de dépôt: **98400211.3**

(22) Date de dépôt: **02.02.1998**

(54) **Composition déodorante comprenant un dendrimère**

Dendrimer enthaltende Deodorantzusammensetzung

Deodorant composition comprising a dendrimer

(84) Etats contractants désignés:
**DE ES FR GB IT**

(30) Priorité: **14.02.1997 FR 9701750**

(43) Date de publication de la demande:
**19.08.1998 Bulletin 1998/34**

(73) Titulaire: **L'OREAL**
**75008 Paris (FR)**

(72) Inventeurs:
• **Forestier, Serge**
**77410 Claye Souilly (FR)**

• **Rollat-Corvol, Isabelle**
**92100 Boulogne (FR)**

(74) Mandataire: **Andral, Christophe André Louis**
**L'OREAL-DPI**
**6 rue Bertrand Sincholle**
**92585 Clichy Cedex (FR)**

(56) Documents cités:
**EP-A- 0 684 044      WO-A-82/01993**
**WO-A-93/14147      WO-A-95/02008**
**WO-A-96/14346      WO-A-96/30002**
**FR-A- 2 368 509**

**Description**

La présente invention concerne une composition déodorante, de préférence une composition cosmétique, comprenant au moins un polymère choisi parmi les dendrimères à groupements terminaux porteurs d'une fonction amine primaire, l'utilisation de ces compositions pour l'application topique humaine et l'utilisation d'un polymère choisi parmi lesdits dendrimères comme actif déodorant.

Elle concerne également un procédé de désodorisation mettant en oeuvre ladite composition et plus spécialement un procédé pour traiter les odeurs axillaires humaines consistant à appliquer sur la surface axillaire une quantité efficace de ladite composition.

Dans le domaine cosmétique, il est bien connu d'utiliser en application topique, des produits déodorants contenant des substances actives de type antitranspirant ou de type bactéricide pour diminuer voire supprimer les odeurs axillaires généralement désagréables.

Les substances antitranspirantes ont pour effet de limiter le flux sudoral. Elles sont généralement constituées de sels d'aluminium qui, d'une part, sont irritants pour la peau et qui, d'autre part, diminuent le flux sudoral en modifiant la physiologie cutanée, ce qui n'est pas satisfaisant.

Les substances bactéricides inhibent le développement de la flore cutanée responsable des odeurs axillaires. Parmi les produits bactéricides, le plus couramment employé est le Triclosan (5-chloro-2-(2,4-dichlorophénoxy) phénol), qui présente l'inconvénient de modifier de façon importante l'écologie de la flore cutanée, d'être inhibé par certains composés, comme par exemple les tensio-actifs non-ioniques, couramment utilisés dans la formulation de compositions cosmétiques. Le caractère insoluble du Triclosan dans l'eau ne permet pas son incorporation dans des formules essentiellement aqueuses.

Dans le but d'obtenir une efficacité à long terme, on recherche de nouveaux produits exerçant une action d'actif déodorant, c'est-à-dire des produits qui soient capables de modifier, de réduire et/ou d'ôter ou de prévenir le développement des odeurs corporelles (cette définition est donnée dans l'ouvrage « Cosmetic Science and Technology Series » - 1988 / Volume 7 chap.10 - IIIc). En outre, on cherche des produits qui ne présentent pas les inconvénients des substances actives utilisées dans l'art antérieur. On sait que certains composés basiques ont la propriété d'absorber les odeurs. C'est le cas par exemple du bicarbonate de sodium et du glycinate de zinc dont on suppose que l'action consiste à neutraliser les acides gras à chaines courtes responsables, parmi d'autres composés, des odeurs axilliaires désagréables.

On sait que certains composés poly-aminés permettent de limiter le développement des mauvaises odeurs. C'est le cas par exemple de la polyéthylène imine (polymère hyperbranché), pour laquelle on pourra se référer à la demande de brevet WO82/01993. Toutefois l'efficacité des molécules polyaminées connues est très insuffisante, en particulier, on constate que leur action est quasi-nulle seulement quelques heures après l'application.

On sait dans les demandes EP 0684044 et WO 96/30002 que certains polymères du type dendrimère sont utilisés en cosmétique pour l'entrappage et le relargage contrôlé de substances actives cosmétiques comme des parfums ou des déodorants.

Après de nombreuses recherches menées sur la question, la demanderesse a découvert, de façon étonnante, que les dendrimères à groupements terminaux porteurs d'une fonction amine primaire présentent la propriété de prévenir le développement des mauvaises odeurs, sans les inconvénients des substances actives antérieurement employées dans les compositions déodorantes, et avec l'avantage d'être hydrosolubles dans des proportions intéressantes et suffisantes pour être aisément formulables, notamment dans les compositions cosmétiques à base d'eau pour l'application topique humaine. Ces compositions sont en outre non-toxiques et non-irritantes.

La présente invention a ainsi pour premier objet une nouvelle composition déodorante comprenant au moins un polymère choisi parmi les dendrimères à groupements terminaux porteurs d'une fonction amine primaire.

Elle a aussi pour objet l'utilisation desdites compositions, plus spécialement comme, ou pour la fabrication de, produits cosmétiques destinés à l'application topique humaine.

Elle a également pour objet l'utilisation d'au moins un polymère choisi parmi les dendrimères à groupements terminaux porteurs d'une fonction amine primaire comme actif déodorant, en particulier comme agent inhibiteur du développement d'odeurs.

Elle a enfin pour objet un procédé de désodorisation mettant en oeuvre ladite composition, et plus particulièrement un procédé pour traiter les odeurs axillaires humaines, consistant à appliquer sur la surface axillaire une quantité efficace de ladite composition.

Les polymères hyperbranchés, catégorie de polymère à laquelle appartient la polyéthylèneimine, sont des contructions moléculaires ayant une structure ramifiée, en général autour d'un coeur. Leur structure est en règle générale dépourvue de symétrie : les unités de base ou monomères ayant servi à la construction du polymère hyperbranché peuvent être de natures différentes et leur répartition est irrégulière. Les branches du polymère peuvent être de natures et de longueurs différentes. Le nombre d'unités de base, ou monomères, peut être différent suivant les différentes ramifications.

Les polymères hyperbranchés sont généralement issus de la polycondensation d'un ou plusieurs monomères ABx, A et B étant des groupements réactifs susceptibles de réagir ensemble, x étant un entier supérieur ou égal à 2, mais d'autres procédés de préparation peuvent être envisagés. Les polymères hyperbranchés se caractérisent par leur degré de polymérisation DP = 1-b, b étant le pourcentage de fonctionnalités, non terminales, de B qui n'ont pas réagi avec un groupement A. La condensation étant non systématique, au contraire de la synthèse de dendrimères, le degré de polymérisation est inférieur à 100%. De façon habituelle, par les méthodes de synthèses connues, DP est compris entre 15 et 90%.

Les dendrimères sont des polymères et oligomères hautement ramifiés, également connus en soi, ayant une structure chimique bien définie et on dit que ce sont des polymères hyperbranchés « parfaits ». En règle générale, les dendrimères comprennent un coeur, un nombre déterminé de générations de branches, ou fuseaux, et des groupes terminaux. Les générations de fuseaux sont constituées d'unités structurelles , qui sont identiques pour une même génération de fuseaux et qui peuvent être identiques ou différentes pour des générations de fuseaux différentes. Les générations de fuseaux s'étendent radialement en une progression géométrique à partir du coeur. Les groupes terminaux d'un dendrimère de la $N^{ième}$ génération sont les groupes fonctionnels terminaux des fuseaux de la $N^{ième}$ génération ou génération terminale. De tels polymères sont décrits en particulier dans D.A.Tomalia, A.M.Naylor et W.A. Goddard III, *Angewandte Chemie,* Int.Ed.Engl.29, 138-175 (1990); C.J.Hawker et J.M.J.Frechet, *J.Am.Chem.Soc.*, 112, 7638 (1990) ; B.I.Voit, Acta Polymer., 46, 87-99 (1995) ; N.Ardoin et D.Astruc, Bull. Soc. Chim. Fr. 132, 875-909 (1995) ; G.R.Newkome, C.N.Moorefield, F.Vögtle, Dendritic Molecules, VCH Verlagsgesellschaft, 1996.

On peut également plus particulièrement définir les dendrimères par la formule (DI) suivante :

$$C[A_1B_1(A_2B_2(...(A_{n-1}\ B_{n-1}(A_nB_n(T)r_n)r_{n-1})r_{n-2}...)r_2)r_1]s \qquad\qquad (DI)$$

dans laquelle :

-   C représente le coeur, relié par un nombre s de fonctionnalités à s fuseaux $A_1B_1$ par l'intermédiaire des groupements $A_1$ ;

-   s est un nombre entier supérieur ou égal à 1 et inférieur ou égal au nombre de fonctionnalités de C ;

-   pour chaque fuseau $(A_iB_i)$ (i=1, 2.....n), le groupement $B_i$ est relié à $r_i$ groupements $A_{i+1}$ d'un fuseau $(A_{i+1}B_{i+1})$ ;

-   chaque groupement $A_i$ (i ≥ 2) est relié à un seul groupement $B_{i-1}$ du fuseau $(A_{i-1}B_{i-1})$ ;

-   $r_i$ (i=1, 2.....n-1) représente le nombre de fonctionnalités du groupement $B_1$ appartenant au fuseau $(A_iB_i)$ ; ri étant un nombre entier supérieur ou égal à 2

-   l'indice i (i=1, 2.....n) est un nombre entier qui désigne la génération de chaque fuseau ;

-   le fuseau de $n^{ième}$ génération $A_nB_n$ est chimiquement lié à un nombre $r_n$ de groupes terminaux T ; $r_n$ étant un entier supérieur ou égal à zéro.

La définition des dendrimères donnée ci-dessus inclut des molécules à ramifications symétriques ; elle inclut également des molécules à ramification non symétrique, comme par exemple les dendrimères dont les fuseaux sont des groupements lysine, dans lesquels le branchement d'une génération, de fuseaux sur la précédente se fait sur les amines a et e de la lysine, ce qui conduit à une différence dans la longueur des fuseaux des différentes ramifications.

Les polymères denses en étoiles, ou « dense star polymer », les polymères éclatés en étoile, ou « starburst polymer », les dendrimères en baguette, ou « rod-shaped dendrimer », sont inclus dans la présente définition des dendrimères. Les molécules dénommées arborols et molécules en cascade entrent également dans la définition des dendrimères selon la présente invention.

Plusieurs dendrimères peuvent être associés entre eux, par une liaison covalente ou un autre type de liaison, par l'intermédiaire de leurs groupes terminaux pour donner des entités connues sous le nom de « dendrimères pontés », « agrégats de dendrimères » ou « bridged dendrimer ». De telles entités sont incluses dans la définition des dendrimères selon la présente invention.

Des dendrimères peuvent se présenter sous la forme d'un ensemble de molécules de même génération, ensembles dits monodisperses ; ils peuvent également se présenter sous la forme d'ensembles de générations différentes, dits polydisperses. La définition des dendrimères selon la présente invention inclut des ensembles monodisperses aussi bien que polydisperses de dendrimères.

L'invention concerne plus particulièrement les dendrimères à groupements terminaux porteurs d'une fonction amine primaire.

On peut se reporter aux documents suivants dans lesquels sont décrits des dendrimères dont le groupe terminal comporte une fonction amine, le contenu de ces documents étant incorporé par référence dans la présente description : US-4,694,064; US-4,507,466 US-4,631,337 ; US-4,558,120 ; US-4,568,737 ; US-4,587,329 ; WO-A-9502008 ; WO-A-9314147 ; EP-234408 ; US-4,289,872 ; US-4.360,646; Proc. Natl. Acad. Sci. USA, 85, 5409-5413 (1988); WO95/02008 ; WO93/14147.

Les dendrimères à groupes terminaux porteurs d'une fonction aminé primaire sont des polyamidoamines, comme par exemple ceux commercialisés sous la marque STARBURST PAMAM par la société DENDRITECH (copolymères séquencés d'éthylène diamine et d'acrylate de méthyle). Ils peuvent également être choisis parmi les dendrimères du type polyalkylamine, comme par exemple les polyéthylèneimines et les polypropylèneimines fabriqués par la société DSM.

Les dendrimères à groupes terminaux porteurs d'une fonction amine primaire peuvent également être constitués d'un coeur et de générations d'unités de base, monomères ou fuseaux, de toutes natures, sur lesquels un groupe terminal T porteur d'une fonction amine a été greffé.

Lorsque le dendrimère utilisé est un polyamidoamine, celui-ci est choisi de préférence parmi ceux de génération 1, génération 2, génération 3, génération 4 et génération 5 et plus particulièrement encore de génération 1.

De préférence, les fonctions amines du dendrimère sont neutralisées. Cette neutralisation permet de conférer à ces molécules à la fois une meilleure tolérance par la peau (respect du pH physiologique) et une meilleure efficacité d'inhibiteur du développement d'odeurs.

Dans les compositions déodorantes selon la présente invention, le dendrimère à groupements terminaux porteurs d'une fonction amine primaire est généralement présent dans des proportions pondérales, calculées en poids par rapport au poids total de la composition, allant environ de 0,01 à environ 10%, de préférence allant environ de 0,1 à environ 5%, et mieux encore allant environ de 0,1 à 2%.

Les compositions déodorantes de la présente invention peuvent comprendre en outre d'autres actifs déodorants classiques en plus des dendrimères définis selon l'invention.

Ces actifs déodorants peuvent être choisis par exemple parmi : Des sels hydrosolubles de zinc, comme par exemple le pyrrolidone carboxylate de zinc (plus communément appelé pidolate de zinc), le sulfate de zinc, le chlorure de zinc, le lactate de zinc, le gluconate de zinc et le phénolsulfonate de zinc ; des sels d'aluminium, comme par exemple le chlorure d'aluminium et les hydroxyhalogénures d'aluminium ; des sels de zirconium, comme par exemple des sels d'oxyde de zirconium, des sels d'hydroxyzirconyle ; des complexes de métaux comme l'aluminium ou le zirconium avec un acide aminé comme par exemple la glycine comme décrit dans US-3,792,068 ; des bactéricides.

Les compositions déodorantes de la présente invention sont formulées classiquement selon les applications auxquelles elles sont destinées.

Lorsqu'elles sont destinées à un usage cosmétique, elles sont plus particulièrement formulées dans un véhicule cosmétiquement acceptable qui peut être notamment essentiellement aqueux, et contenir des monoalcools en $C_1$-$C_4$, de préférence de l'éthanol pour accélérer l'évaporation du produit. Les monoalcools sont généralement présents en quantités allant de 15 à 50%, préférentiellement de 20 à 45% et encore plus préférentiellement de 25 à 35%.

Les compositions selon l'invention peuvent également être formulées en émulsion eau-dans-huile, huile-dans-eau, ou en émulsion triple eau-dans-huile-dans-eau, (de telles émulsions sont connues et décrites par exemple par C. FOX dans « Cosmetics and Toiletries » - november 1986 - Vol 101 - pages 101-112), auquel cas le dendrimère est présent dans la phase aqueuse de l'émulsion.

Les compositions de l'invention peuvent comprendre en outre des adjuvants cosmétiques choisis parmi les corps gras, les solvants organiques, les gélifiants, les émollients, les adoucissants, les antioxydants, les opacifiants, les stabilisants, les silicones, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, les parfums, les colorants, les pigments, les agents épaississants, ou tout autre ingrédient habituellement utilisé en cosmétique.

Les tensio-actifs sont choisis de préférence parmi les tensio-actifs non-ioniques, comme par exemple les produits de condensation d'un alcool gras ou d'un acide gras avec une chaine polyalkylèneglycol.

Les corps gras peuvent être constitués par une huile ou une cire ou leur mélange, la vaseline, la paraffine, la lanoline, la lanoline hydrogénée, la lanoline acétylée ; ils comprennent également les acides gras, les alcools gras tels que l'alcool laurique, cétylique, myristique, stéarique, palmitique, oléique ainsi que le 2-octyldodécanol, les esters d'acides gras tels que le monostéarate de glycérol, le monostéarate de polyéthylèneglycol, le myristate d'isopropyle, l'adipate d'isopropyle, le palmitate d'isopropyle, le palmitate d'octyle, les benzoates d'alcools gras en $C_{12}$-$C_{15}$ (Finsolv TN de FINETEX), l'alcool myristique polyoxypropyléné à 3 moles d'oxyde de propylène (WITCONOL APM de WITCO), les triglycérides d'acides gras en $C_6$-$C_{18}$ tels que les triglycérides d'acide caprylique/caprique.

Les huiles sont choisies parmi les huiles animales, végétales, minérales ou de synthèse et notamment l'huile de palme hydrogénée, l'huile de ricin hydrogénée, l'huile de vaseline, l'huile de paraffine, l'huile de Purcellin (octanoate de stéaryle), les huiles de silicone et les isoparaffines.

Les cires sont choisies parmi les cires animales, fossiles, végétales, minérales ou de synthèse. On peut citer notamment les cires d'abeille, les cires de Carnauba, de Candelila, de canne à sucre, du Japon, les ozokérites, la cire de Montan, les cires microcristallines, les paraffines, les cires et résines de silicone.

Les épaississants, de préférence non ioniques, peuvent être choisis parmi les gommes de guar et celluloses modifiées ou non telles que la gomme de guar hydroxypropylée, la cétylhydroxyéthylcellulose, les silices comme par exemple la Bentone Gel MiO vendue par la société NL INDUSTRIES ou la Veegum Ultra, vendue par la société POLYPLASTIC.

Elles peuvent comprendre des émollients, qui contribuent à une sensation douce, sèche, non-collante à l'application de la composition sur la peau. Ces émollients peuvent être choisis parmi des produits du type silicone volatile, des silicones non-volatiles et d'autres émollients non-volatils. Généralement les émollients sont présents en proportions allant de 20 à 80%, de préférence de 25 à 60% et encore plus préférentiellement de 35 à 55%.

Les silicones volatiles sont définies de façon connue comme des composés volatils à température ambiante. On peut citer parmi ces composés les silicones volatiles cycliques et linéaires du type diméthylsiloxane dont les chaines comprennent de 3 à 9 résidus siliconés. De préférence on choisit les cyclométhicones D4 ou D5.

Les silicones non-volatiles sont définies de façon connue comme des composés de pression de vapeur faible à température ambiante. Parmi ces composés sont inclus: les polyalkylsiloxanes, en particulier les polyalkylsiloxanes linéaires comme par exemple les polydiméthylsiloxanes, ou diméthicones, linéaires, commercialisés par la société Dow Corning sous le nom de « Dow Corning 200 Fluid » ; les polyalkylarylsiloxanes comme par exemple les polyméthylphénylsiloxanes, commercialisés par la société Dow Corning sous le nom de « Dow Corning 556 Fluid » ; les copolymères polyéther et siloxane, comme par exemple les diméthicone copolyols.

Parmi les émollients non-volatils utilisables dans la présente invention, on peut citer par exemple : les dérivés hydrocarbonés, les huiles minérales, les alcools gras, les esters d'alcools en $C_3$-$C_{18}$ avec des acides en $C_3$-$C_{18}$, les esters de l'acide benzoïque avec des alcools en $C_{12}$-$C_{13}$ et leurs mélanges, des polyols en $C_2$-$C_6$ choisis de préférence parmi le glycérol, le propylèneglycol ou le sorbitol, les polymères de polalkylène glycol.

Lorsque les compositions déodorantes selon l'invention sont destinées à l'usage cosmétique, elles peuvent se présenter sous forme de lotions, de crèmes ou de gels fluides distribués en spray aérosol, en flacon pompe ou en roll-on, sous forme de crèmes épaisses distribuées en tubes et sous forme de sticks, et contenir à cet égard les ingrédients et propulseurs généralement utilisés dans ce type de produits et bien connus de l'homme de l'art, sous réserve qu'ils n'interfèrent pas avec les dendrimères décrits dans la présente invention.

L'invention peut aussi trouver des applications intéressantes dans le domaine des produits d'entretien et désodorisants divers (air ambiant, textiles, réfrigérateurs, vide-ordures, poubelles, litières et cages d'animaux domestiques ou gaines de ventilation des immeubles).

Des exemples concrets, mais nullement limitatifs, illustrant l'invention vont maintenant être donnés.

ESSAI 1

On a réalisé un test d'inhibition du développement d'odeurs sur de la sueur naturelle.

On a donc prélevé en sauna les sueurs axillaires de plusieurs sujets humains que l'on a réunies pour réaliser un échantillon de sueur. Dans chaque flacon de substance active à tester ainsi que dans un flacon témoin (sans substance active), on a introduit 1 ml de l'échantillon de sueur incubée. Puis on a introduit la substance active : x x mg (x=1 ,10 ,20) de dendrimère choisis parmi les STARBURST PAMAM DENDRIMER de génération 1, 2, 3 et 5, (désignés PAMAM GEN 1 à 5) commercialisés par la société DENDRITECH, respectivement dans chacun des flacons de substance active à tester.

On a ensuite procédé à une incubation des flacons à la température de 37°C. 4 à 6 évaluateurs ont ensuite senti (« sniff-test ») plusieurs fois à quelques heures d'intervalle chacun de ces flacons et noté l'intensité de l'odeur sur une échelle de 0 à 4.

Note 0 = pas d'odeur.

Note 4 = odeur forte.

Résultats :

Les résultats de ces essais sont consignés dans le tableau 1 :

Tableau 1

| Essai | Actif | Quantité d'actif (mg d'actif par ml de sueur) | Note d'odeur à t=0h | Note d'odeur à t=20h | Note d'odeur à t=24h |
|---|---|---|---|---|---|
| 1.1 | PAMA | 1,8 | 0,3 | 0,3 | 0,7 |
| | M GEN | 17,9 | 0,4 | 0,2 | 0,5 |
| | 1 | 35,9 | 1 | 0,5 | 0,3 |
| 1.2 | PAMA | 2 | 0,2 | 1,6 | 2,5 |
| | M GEN | 20 | 0,7 | 0,4 | 0,5 |
| | 2 | 40 | 0,7 | 0,5 | 0 |
| 1.3 | PAMA | 2,1 | 0,3 | 0,6 | 2,7 |
| | M GEN | 20,9 | 0,1 | 1,7 | 2,5 |
| | 3 | 41,7 | 0,9 | 0,4 | 2,3 |
| 1.4 | PAMA | 2,3 | 0,4 | 1,3 | 2,5 |
| | M GEN | 22,7 | 0,3 | 2 | 2,3 |
| | 5 | 45,4 | 0,7 | 0,5 | 2 |
| 1.5 | - | 0 | 0,1 | 2,5 | 2,7 |

Ces résultats démontrent que les dendrimères présentent une importante capacité d'inhibition du développement des odeurs de la sueur naturelle pendant au moins 20 heures et jusqu'à au moins 24 heures pour les dendrimères des 1ère et 2ème générations.

ESSAI 2

On a comparé la capacité des dendrimères à prévenir le développement d'odeurs par rapport à d'autres bases. On a suivi le même protocole qu'à l'exemple 1 en utilisant comme actifs déodorants :

- un dendrimère, le STARBURST PAMAM DENDRIMER de génération 1 à un taux de 1mg par ml de sueur, ce qui donne un pH de la solution à tester de 8,9 ;
- de la polylysine, commercialisée par la société CHISSO sous le nom de POLYLYSINE, incorporé à la sueur en quantité suffisante pour avoir un pH égal à 8,9 ;
- de la soude, incorporé à la sueur en quantité suffisante pour avoir un pH égal à 8,9.

Les résultats de ce test sont consignés dans le tableau 2 :

Tableau 2

| Essai | Actif | pH initial | Note d'odeur à t=0h | Note d'odeur à t=20h | Note d'odeur à t=24h |
|---|---|---|---|---|---|
| 2.1 | PAMAM GEN 1 | 8,9 | 0 | 0,8 | 1,8 |
| 2.2 | Polylysine | 8,9 | 0 | 2,6 | 2,8 |
| 2.3 | NaOH | 8,9 | 0 | 3,6 | 2,8 |
| 2.4 | - | 7,8 | 0 | 3,1 | 3,1 |

Ces essais montrent la supériorité des dendrimères sur les amines connues de l'art antérieur, en particulier sur un polymère poly-aminé linéaire comme la polylysine.

ESSAI 3 :

On a comparé la capacité des dendrimères à inhiber le développement des odeurs par rapport à d'autres

## EP 0 858 797 B1

bases. On a suivi le même protocole qu'à l'exemple 1 en utilisant comme actifs déodorants :

- un dendrimère, le STARBURST PAMAM DENDRIMER de génération 1 à un taux de 1mg par ml de sueur, ce qui donne un pH de la solution à tester de 8,8;
- de la polyéthylèneimine (polymère hyperbranché) commercialisée par la société BASF sous le nom de Polymin G-35, incorporé à la sueur en quantité suffisante pour avoir un pH égal à 8,8.

Les résultats de ces tests sont consignés dans le tableau 3 :

Tableau 3

| Essai | Actif | pH initial | Note d'odeur t=14h | Note d'odeur à à t=20h | Note d'odeur à t=24h |
|---|---|---|---|---|---|
| 3.1 | PAMAM GEN 1 | 8,8 | 0,1 | 0,8 | 0,3 |
| 3.2 | Polyéthylèn eimine | 8,8 | 0,7 | 1,7 | 0,7 |
| 3.3 | - | 8,2 | 1,9 | 1,8 | 2,3 |

ESSAI 4 :

Afin de se rapprocher des conditions d'une formulation respectant le pH physiologique cutané, un test d'efficacité des actifs a été fait à pH7.

On a donc suivi le même protocole que décrit ci-dessus sur deux sujets distincts, avec une seule évaluation à 20h, en utilisant des solutions sueur+actif neutralisées par HCl pour obtenir un pH égal à 7. On a donc testé :

4.1- un dendrimère, le STARBURST PAMAM DENDRIMER de génération 1 à un taux de 1mg par ml de sueur, ce qui donne un pH de la solution à tester de 8,9 ;
4.2- la solution 4.1 neutralisée par HCl ;
4.3- un dendrimère, le STARBURST PAMAM DENDRIMER de génération 5 à un taux de 1mg par ml de sueur, ce qui donne un pH de la solution à tester de 8,9 ;
4.4- la solution 4.3 neutralisée par HCl ;
4.5- la solution témoin : pas d'actif, pas de neutralisation

Les résultats de ces essais sont consignés dans le tableau 4 :

Tableau 4

| Essai | Actif | pH initial | Note d'odeur | Note d'odeur |
|---|---|---|---|---|
| 4.1 | PAMAM GEN 1 | 8,8 | 0,8 | 1 |
| 4.2 | PAMAM GEN 1 | 7 | 0,3 | 0,5 |
| 4.3 | PAMAM GEN 5 | 8,8 | 1 | 1,3 |
| 4.4 | PAMAM GEN 5 | 7 | 0,5 | 0,3 |
| 4.5 | - | 8,2 | 2,1 | 2,6 |

Ces résultats montrent la supériorité des dendrimères neutralisés par rapport aux dendrimères non neutralisés.

EXEMPLES

On a réalisé les quatre formules suivantes (deux lotions aqueuses, un stick aqueux et une émulsion huile-dans-eau):

1- Lotion aqueuse :

- STARBURST PAMAM DENDRIMER de génération 1      0,5 %
- Parfum      qs
- Eau déminéralisée      qsp 100

2- Lotion aqueuse :

- STARBURST PAMAM DENDRIMER de génération 1      0,5 %
- Parfum      qs
- Conservateur      qs
- HCl      qs pH7
- Eau déminéralisée      qsp 100

3- Stick aqueux :

On a réalisé un stick aqueux déodorant de composition suivante :

- Stéarate de sodium      6,2 %
- Glycérine      15 %
- Propylène glycol      20 %
- STARBURST PAMAM DENDRIMER de génération 1      1,8 %
- Parfum      qs
- Eau déminéralisée      qsp 100

4- Crème déodorante :

On a réalisé une crème déodorante sous la forme d'une émulsion huile dans eau de composition suivante :

Phase huileuse :

- Mélange d'alcools cétylstéarylique et cétylstéarylique oxyéthylénés (330E) vendu sous la dénomination DEHSCONET 390 par la société TENSIA      7 %
- Stéarate de glycérol (mono- et di-) vendu sous la dénomination GELEOL COPEAUX par la société GATTEFOSSE      2 %
- Alcool cétylique      1,5 %
- Polydiméthysiloxane vendu sous la dénomination SILBIONE HUILE 70 047 V300 par la société RHONE POULENC      1,5 %
- Huile de vaseline      15 %
- Glycérine      20 %

Phase aqueuse :

- STARBURST PAMAM DENDRIMER de génération 1      1,8 %
- Propylène glycol      20 %
- Parfum      qs
- Conservateurs      qs
- HCl      qs pH7
- Eau déminéralisée      qsp 100

Cette crème est efficace pour inhiber le développement des odeurs axillaires humaines.

**Revendications**

1. Utilisation dans une composition cosmétique d'au moins un polymère choisi parmi les dendrimères dont les groupements terminaux sont porteurs d'une fonction amine primaire, comme actif déodorant.

2. Utilisation dans une composition cosmétique d'au moins un polymère choisi parmi les dendrimères dont les groupements terminaux sont porteurs d'une fonction amine primaire, comme agent inhibiteur du développement d'odeurs.

3. Utilisation selon la revendication 1 ou 2, **caractérisée par le fait que** le dendrimère est choisi parmi les polyamidoamines et les polyalkylamines.

4. Utilisation selon la revendication 3, **caractérisée par le fait que** le dendrimère est choisi parmi les polyamidoamines de 1ère génération.

5. Utilisation selon l'une quelconque des revendications 1 à 4, **caractérisée par le fait que** les fonctions aminés des groupes terminaux du dendrimère sont neutralisées.

6. Composition cosmétique déodorante sous forme d'une lotion, d'une crème ou d'un gel fluide distribués en spray aérosol, en flacon pompe ou en roll-on, sous forme d'une crème épaisse distribuée en tubes et sous forme de stick, **caractérisée par le fait qu'**elle comprend au moins un polymère tel que défini dans l'une quelconque des revendications 1 à 5.

7. Composition selon la revendication 6, **caractérisée par le fait que** le dendrimère est présent dans des proportions allant de 0,01 à 10 % en poids par rapport au poids total de la composition.

8. Composition selon la revendication 7, **caractérisée par le fait que** le dendrimère est présent dans des proportions allant de 0,1 à 5%, et préférentiellement de 0,1 à 2%, en poids par rapport au poids total de la composition.

9. Composition selon l'une quelconque des revendications 6 à 8, **caractérisée par le fait qu'**elle comprend en outre d'autres actifs déodorants.

10. Composition selon l'une quelconque des revendications 6 à 9, **caractérisée par le fait qu'**elle est formulée dans un véhicule essentiellement aqueux.

11. Composition selon l'une quelconque des revendications 6 à 10, **caractérisée par le fait qu'**elle est formulée en émulsion eau-dans-huile, huile-dans-eau, ou en émulsion triple eau-dans-huile-dans-eau,

12. Procédé cosmétique pour traiter les odeurs axillaires humaines, consistant à appliquer sur la surface axillaire une quantité efficace d'une composition selon l'une quelconque des revendications 6 à 10.

**Patentansprüche**

1. Verwendung mindestens eines Polymers, das unter den Dendrimeren ausgewählt ist, deren endständige Gruppen eine primäre Aminogruppe tragen, in kosmetischen Zusammensetzungen als desodorisierender Wirkstoff.

2. Verwendung mindestens eines Polymers, das unter den Dendrimeren ausgewählt ist, deren endständige Gruppen eine primäre Aminogruppe tragen, in kosmetischen Zusammensetzungen als Mittel zur Inhibierung der Geruchsentwicklung.

3. Verwendung nach Anspruch 1 oder 2, **dadurch gekennzeichnet, dass** das Dendrimer unter den Polyamidoaminen und Polyalkylaminen ausgewählt ist.

4. Verwendung nach Anspruch 3, **dadurch gekennzeichnet, dass** das Dendrimer unter den Polyamidoaminen der 1. Generation ausgewählt ist.

**5.** Verwendung nach einem der Ansprüche 1 bis 4, **dadurch gekennzeichnet, dass** die Aminogruppen der endständigen Gruppen des Dendrimers neutralisiert sind.

**6.** Kosmetische desodorisierende Zusammensetzung, die in Form einer Lotion, einer Creme oder eines fluiden Gels, die als Aerosolspray, Pumpflakon oder Roll-on-Deo vertrieben werden, in Form einer dickflüssigen Creme, die in Tuben im Handel ist, und in Form eines Stiftes vorliegt, **dadurch gekennzeichnet, dass** sie mindestens ein Polymer nach einem der Ansprüche 1 bis 5 enthält.

**7.** Zusammensetzung nach Anspruch 6, **dadurch gekennzeichnet, dass** das Dendrimer in Mengenanteilen von 0,01 bis 10 Gew.-%, bezogen auf das Gesamtgewicht der Zusammensetzung, vorliegt.

**8.** Zusammensetzung nach Anspruch 7, **dadurch gekennzeichnet, dass** das Dendrimer in Mengenanteilen von 0,1 bis 5 Gew.-% und vorzugsweise 0,1 bis 2 Gew.-%, bezogen auf das Gesamtgewicht der Zusammensetzung, vorliegt.

**9.** Zusammensetzung nach einem der Ansprüche 6 bis 8, **dadurch gekennzeichnet, dass** sie außerdem weitere desodorisierende Wirkstoffe enthält.

**10.** Zusammensetzung nach einem der Ansprüche 6 bis 9, **dadurch gekennzeichnet, dass** sie in einem im Wesentlichen wässrigen Träger formuliert ist.

**11.** Zusammensetzung nach einem der Ansprüche 6 bis 10, **dadurch gekennzeichnet, dass** sie als Wasser-in-Öl-Emulsion, Ölin-Wasser-Emulsion oder dreifache Wasser-in-Öl-in-Wasser-Emulsion formuliert ist.

**12.** Kosmetisches Verfahren, um Gerüchen im menschlichen Achselbereich entgegenzuwirken, das darin besteht, eine wirksame Menge einer Zusammensetzung nach einem der Ansprüche 6 bis 10 in der Achselhöhle aufzubringen.

**Claims**

**1.** Use, in a cosmetic composition, of at least one polymer chosen from dendrimers in which the terminal groups bear a primary amine function, as a deodorant active agent.

**2.** Use, in a cosmetic composition, of at least one polymer chosen from dendrimers whose end groups bear a primary amine function, as an agent for inhibiting the development of odours.

**3.** Use according to Claim 1 or 2, **characterized in that** the dendrimer is chosen from polyamidoamines and polyalkylamines.

**4.** Use according to Claim 3, **characterized in that** the dendrimer is chosen from 1st generation polyamidoamines.

**5.** Use according to any one of Claims 1 to 4, **characterized in that** the amine functions in the terminal groups of the dendrimer are neutralized.

**6.** Cosmetic deodorant composition in the form of a lotion, a cream or a fluid gel distributed in an aerosol spray, a pump-dispenser bottle or a roll-on, in the form of a thickened cream distributed in tubes and in the form of a stick, **characterized in that** it comprises at least one polymer as defined in any one of Claims 1 to 5.

**7.** Composition according to Claim 6, **characterized in that** the dendrimer is present in proportions ranging from 0.01 to 10% by weight relative to the total weight of the composition.

**8.** Composition according to Claim 7, **characterized in that** the dendrimer is present in proportions ranging from 0.1 to 5% by weight, and preferably from 0.1 to 2% by weight, relative to the total weight of the composition.

**9.** Composition according to any one of Claims 6 to 8, **characterized in that** it also comprises other deodorant active agents.

10. Composition according to any one of Claims 6 to 9, **characterized in that** it is formulated in an essentially aqueous vehicle.

11. Composition according to any one of Claims 6 to 10, **characterized in that** it is formulated as a water-in-oil or oil-in-water emulsion or as a water-in-oil-in-water triple emulsion.

12. Cosmetic process for treating human underarm odours, which consists in applying an effective amount of a composition according to any one of Claims 6 to 10 to the underarm area.